# EUROPEAN PATENT SPECIFICATION

(11) **EP 0 821 679 B1**
(45) Date of publication and mention of the grant of the patent: **11.07.2001**
(21) Application number: 96910126.0
(22) Date of filing: 19.04.1996
(51) Int. Cl.: C07D 303/32, C07D 303/48, C07D 407/06, C07D 301/12, C07D 407/14

(54) **ASYMMETRIC EPOXIDES, THEIR SYNTHESIS AND USE**
ASYMMETRISCHE EPOXIDE, IHRE SYNTHESE UND IHRE VERWENDUNG
EPOXYDES ASYMETRIQUES, LEUR SYNTHESE ET UTILISATION

(30) Priority: 20.04.1995 GB 9508098; 22.12.1995 GB 9526379
(43) Date of publication of application: 04.02.1998
(73) Proprietor: Chirotech Technology Limited, Cambridge CB4 4WE (GB)
(72) Inventor: ROBERTS, Stanley, Michael ; Dept. of Chemistry, Liverpool L69 3BX (GB); ADGER, Brian, Michael, Great Shelford, Cambridge CB2 5JE (GB)
(74) Representative: Perry, Robert Edward
(86) International application number: GB9600953
(87) International publication number: WO9633183

(56) References cited:
- EP-A- 0 181 475
- EP-A- 0 403 252
- WO-A-91/13066
- GB-A- 1 156 531
- TETRAHEDRON LETTERS, vol. 31, no. 45, 21 October 1990, OXFORD GB, pages 6501-4, XP002006755 P.W. BAURES ET AL.: "An efficient asymmetric synthesis of substituted phenyl glycidic esters" cited in the application
- JOURNAL OF THE CHEMICAL SOCIETY, PERKIN TRANSACTIONS I, LONDON GB, pages 1317-24, XP002006756 S. JULI ET AL.: "Synthetic enzymes. Part 2. Catalytic asymmetric epoxidation by means of polyamino-acids in a triphase system" cited in the application

## Description

### Field of the Invention

This invention relates to epoxides, their synthesis by the asymmetric epoxidation of enones, and their use.

### Background of the Invention

The enantioselective epoxidation of prochiral alkenes is a valuable methodology, which enables two stereogenic centres to be created in a single synthetic operation. Established methods tend to be limited to specific classes of substrate. The best known is the titanium tartrate-catalysed epoxidation of allylic alcohols, which was first reported by Sharpless as a stoichiometric method, in Katsuki *et al*, J. Am. Chem. Soc. (1980) 102:5974, and later adapted into a catalytic variant; see Gao *et al*, J. Am. Chem. Soc (1987) 109:5765.

More recently, epoxidations employing chiral (salen)Mn(III) catalysts have been applied to a variety of alkene substrates, both unfunctionalised and functionalised; see Jacobsen, chapter 4.2 in Catalytic Asymmetric Synthesis, ed. I. Ojima (1993) VCH, New York.

Although both these known processes are proven as generic methodologies for laboratory-scale synthesis, reliance on metal-based catalysts and reagents means that operation on a large scale can be disadvantageous in terms of cost, work-up procedure and effluent disposal.

A third and potentially more economical methodology is the use of metal-free synthetic polypeptides such as poly-L-leucine as catalysts for the asymmetric epoxidation of prochiral α,β-unsaturated ketones of the general formula

R¹R²C=CR³-CO-X (II)

to give the corresponding optically-enriched epoxides

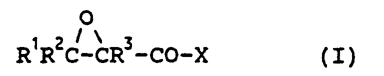

This process was first reported by Juliá *et al*, Angew. Chem. Int. Ed. Engl. (1980) 19:929. However, it is reported that high enantioselectivities are confined to *trans*-chalcone derivatives; see Juliá *et al*, J. Chem. Soc., Perkin Trans. 1 (1982) 1317; Colonna *et al*, Tetrahedron (**1983**) 39:1635; Banfi *et al*, Tetrahedron (1984) 40:5297; Baures *et al*, Tetrahedron Lett. (1990) 31:6501; and Itsuno *et al*, J. Org. Chem. (1990) 55:5047. Thus, this reaction has been considered to be of restricted scope in organic synthesis.

Optically-enriched epoxides are especially suited to nucleophilic ring-opening reactions to give, in stereocontrolled fashion, products bearing heteroatom functionality on adjacent chiral centres. In this respect, (2*R*,3*S*)*-syn*-3-phenylisoserine synthons are reported by Boa *et al*, Contemporary Organic Synthesis (1994) 1:47, and references therein. Several methods proceed via *trans-* or *cis*-phenylglycidate intermediates, prepared by enantioselective oxidation (epoxidation and dihydroxylation) of styrene derivatives; see Greene, J. Org. Chem. (1990) 55:1957; Jacobsen, J. Org. Chem. (1992) 57:4320; and Sharpless, J. Org. Chem. (1994) 59:5105. Although this is an effective overall strategy, provision of enantiopure phenylglycidates relies on the metal-based epoxidation methodologies described above, and aspects of the downstream chemistry are not well suited to operation on a large scale.

Compounds of formula I are known in racemic form. For example, compounds wherein R¹ is phenyl, R² and R³ are each H, and X is t-butyl or cyclopropyl, are disclosed in EP-A-0336841 and WO-A-0113066, and by Matano, J. Chem. Soc. Perkin Trans. I (1994) 2703, Meth-Cohn, *ib.* 1517, and Treves, JACS (1967) 89:6257. The nature of the functional groups makes such compounds difficult to separate into constituent enantiomers, by conventional resolution techniques.

An optically-enriched epoxide of formula I (R¹ = CF₃, R² = R³ = H, X = t-butyl) is reported by Lin *et al*, J. Fluorine Chem. (1989) 44:113-120. Its synthesis is from optically-enriched 1,1,1-trifluoro-2-hydroxy-5,5-dimethylhexan-4-one, using lithium diisopropylamide. This is not a commercial process.

Corey *et al*, Tetrahedron Lett. (1991) 32:2857, report the t-butyl glycidate **5** (see Scheme 1) as the product of a chiral Darzens reaction between t-butyl bromoacetate and benzaldehyde.

### Summary of the Invention

It has surprisingly been discovered that asymmetric epoxidation of the type reported by Juliá *et al* can tolerate a greater range of substituents than is indicated by the prior art. More particularly, the present invention enables the preparation of novel optically-enriched epoxides of formula I wherein R¹, R² and R³ are each independently selected from H, R, R-CO- and R-O-CO-, each R independently being substantially a hydrocarbon group of up to 20 carbon atoms, and X is an alkyl or cycloalkyl group of up to 10 carbon atoms, provided that -CO-X is not enolisable. Novel epoxides of formula I constitute a further aspect of the invention, i.e. wherein the enantiomeric excess is more than 80%, and R¹ is aryl or heteroaryl, optionally linked via an epoxide group to CR².

### Description of the Invention

The nature of each of R¹, R² and R³ is not critical, providing that it does not interfere with the asymmetric epoxidation reaction. For example, R¹ is essentially a spectator to the reaction, for which the non-enolisable nature of -CO-X is important. R¹ may be, for example, a group of up to 10 carbon atoms. R² and R³, and optionally also R¹, may be H. R² and R³ may be linked, e.g. together are -(CH₂)₄-. Any group other than H may comprise C and H atoms only, or may comprise one or more heteroatoms and/or substituents. One preference for R¹ is aryl or heteroaryl, optionally linked via a conjugating group to CR², e.g. phenyl (or substituted phenyl).

Most preferably, X is t-butyl (for example when R¹ is phenyl and R² and R³ are each H), since the substrates for epoxidation are readily available, or simply obtainable from inexpensive readily-available starting materials such as pinacolone. Another simple non-enolisable group is provided when X is cyclopropyl.

X as alkyl can readily be converted to alkoxy by the Baeyer-Villiger reaction. As indicated below, compounds in which X is t-butoxy are of particular interest.

The present invention provides, for example, an asymmetric route to α,β-unsaturated esters or other carboxylates such as amides, in order to access, for instance, the phenylisoserine component of the anticancer natural product taxol or intermediates for the antihypertensive drug diltiazem (for which R¹ is 4-methoxyphenyl).

Obviously a benefit of the methodology is that either enantiomer of the epoxide can be obtained with equal facility by using the appropriate catalyst, e.g. either the L- or the D-polyamino-acid. Other catalysts can be used, as may be found effective, by trial and error. The catalyst may be a material obtainable by nucleophile-promoted oligomerisation of an amino-acid carboxy anhydride. An alternative catalyst is the immobilised catalyst system described by Itsonu *et al*, J. Org. Chem. (1990).

The conditions reported by Juliá *et al, supra,* for the asymmetric epoxidation comprise a three-phase system of poly-amino-acid catalyst, an organic solvent such as n-hexane, and an aqueous phase containing a large excess of both oxidant (hydrogen peroxide) and alkali (sodium hydroxide). For the economic utilisation of the methodology for the manufacture of bulk single-enantiomer intermediates to, e.g. pharmaceuticals, it would be desirable to reduce the need for any excesses of reagents.

It has been discovered that, by the use of solutions of perborates, the amount of alkali, e.g. hydroxide, required in the reaction can be cut down substantially. As a result, apart from the saving in reagents, substrates may be used that are otherwise sensitive to the high alkali concentrations present. In the novel conditions, the oxidation system comprises the poly-amino-acid catalyst, an organic solvent such as dichloromethane, and an aqueous phase containing (sodium) perborate and alkali, e.g. sodium hydroxide. In addition, some phase transfer catalyst such as Aliquat 336 is added. A discovery is that only one equivalent of the sodium hydroxide is required. These oxidation conditions may apply to other heterogeneous oxidations.

Scheme 1 below illustrates reactions according to this invention, shows useful embodiments **1**, **2** and **3**, and an important, illustrative use of products of this invention. All these embodiments may be generalised to the scope of the invention.

The following Examples illustrate the invention.

As shown in Table 1, a variety of epoxyketones (R² = R³ = H) have been prepared in good to excellent yield and excellent optical purity (Example A is for comparison). All these epoxidations were carried out at ambient temperature in a three-phase system with an organic solvent, a catalytic amount of poly-L-leucine synthesised according to Flisak *et al*, J. Org. Chem. (1993) 58:6247, or poly-D-leucine synthesised in the same way from D-leucine, and with a large excess of oxidant. Preactivation of the catalyst, by stirring the mixture for 6 hours before addition of the α,β-unsaturated ketone, resulted in a shorter reaction time, e.g. of 1 to 3 days. Preferred solvents for these reactions are hydrocarbons such as hexane or chlorinated solvents such as dichloromethane. Optical purities (as given in Table 1) were as determined by HPLC on a Chiralpac AD column, and absolute configurations assigned as [2*R*,3*S*] for those epoxides obtained from using poly-L-leucine. The catalyst could be recovered and reused.

**Table 1**

| Ex. | R¹ | X | Conditions | Yield (%) | ee (%) |
|---|---|---|---|---|---|
| A | Ph | isopropyl | (i) | | 62 |
| 1 | Ph | t-butyl | (i) | 92 | >98 |
| 2 | Ph | t-butyl | (ii) | 90 | >86 |
| 3 | 2-naphthyl | cyclopropyl | (ii) | 61 | 90 |
| 4 | Ph-CH=CH- | cyclopropyl | (i) | 73 | 74 |

| | | | | | |
|---|---|---|---|---|---|
| Conditions (i) = poly-L-leucine/H₂O₂/NaOH/CHCl₂ | | | | | |
| (ii) = poly-D-leucine/H₂O₂/NaOH/CHCl₂ | | | | | |

The results summarised in Table 1 show that the epoxidation reaction has a broad substrate specificity and is therefore not restricted to chalcones. Satisfactory enantioselectivities were obtained, including a case where the substrate has a second conjugated double bond (Example 4). Example A was a relatively prolonged reaction, by comparison with Examples 1 and 2, owing to the possibility of enolisation.

With reference to Scheme 1, as exemplification of the value of epoxyketones **1**, a further aspect of the present invention is the use of (1*S*,2*R*)-1,2-epoxy-4,4-dimethyl-1-phenyl-3-pentanone **2** (synthon 3 is an alternative) in processes for the preparation of taxol side-chain synthons such as (*2R*, *3S*)-*N*-benzoyl-3-phenylisoserine synthons **4**, wherein the t-butyl ketone functionality serves as a masked carboxylate. As summarised in Scheme 1, conversion of **2** to **4** can be accomplished by either of the following sequences:
(a) Baeyer-Villiger oxidation to produce *t*-butyl 2,3-epoxy-3-phenylpropanoate **5**, inversion of configuration at C-3 to afford cis-epoxide **6**, nucleophilic ring opening at the benzylic position with either ammonia or azide anion (followed by reduction to the amine), *N*-benzoylation and optional acid-catalysed deesterification. In contrast to similar prior art processes for the corresponding n-alkyl ester (McChesney, Tetrahedron: Asymmetry (1994) 5:1683; Jacobsen, J. Org. Chem. (1992) 57:4320), during epoxide ring opening with ammonia the t-butyl ester provides effective protection against unwanted amidation at C-1, and allows final unmasking of the C-1 carboxyl group to be carried out under mild, non-hydrolytic conditions.
(b) Similar to (a), but with Baeyer-Villiger oxidation carried out at the penultimate stage. By this route the *t*-Bu ketone provides effective masking for the C-1 carboxyl group through much of the synthesis.

Scheme 2 shows another use for a compound of the invention, i.e. in the synthesis of an α-hydroxyester precursor to *L*-2-naphthylalanine.

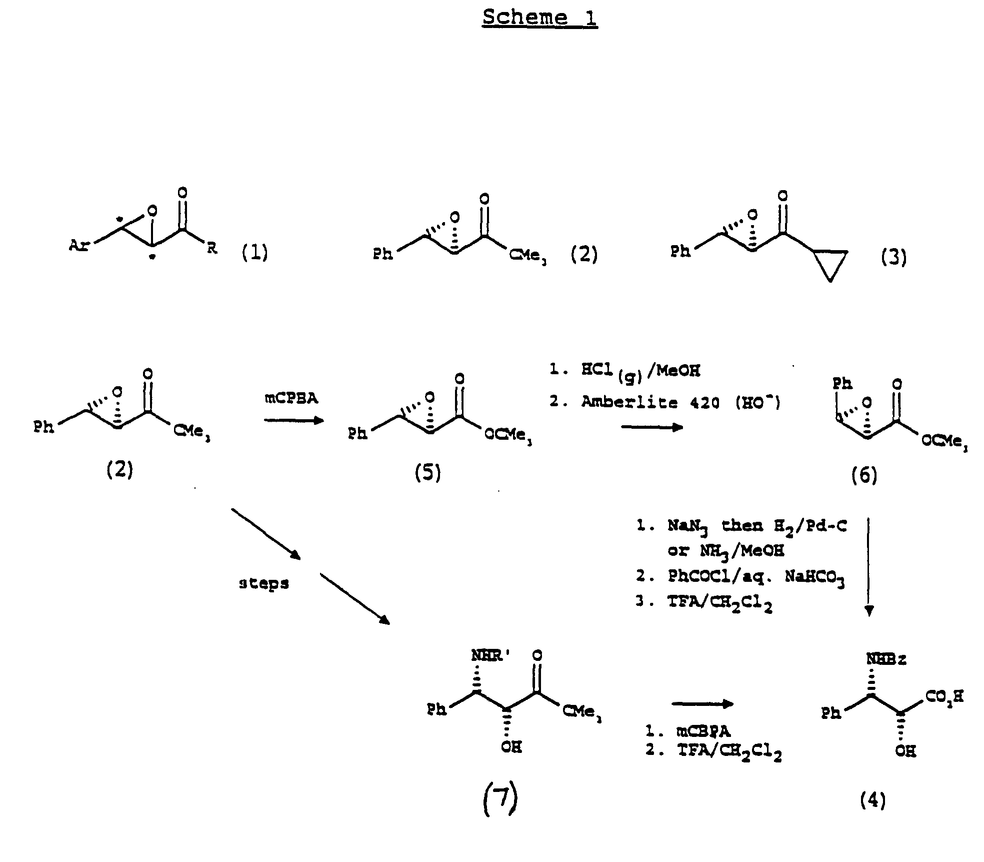

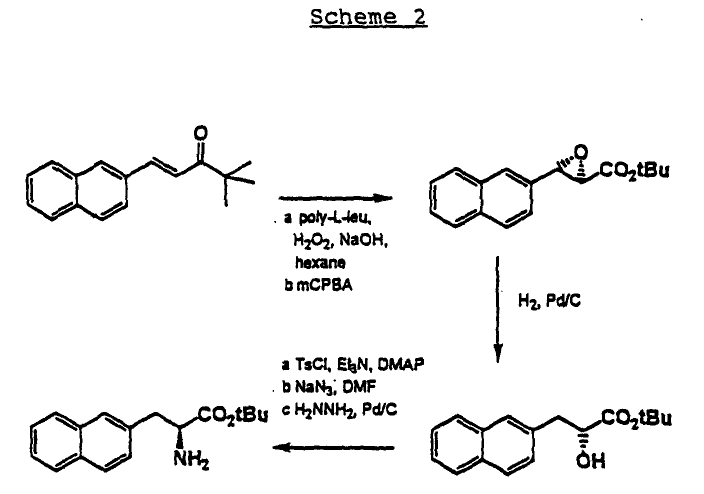

## Claims

1. A process for preparing an optically-enriched chiral epoxide of formula I 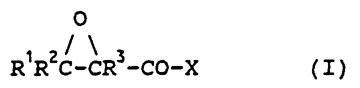 wherein R¹, R² and R³ are each independently selected from H, R, R-CO- and R-O-CO-, each R independently being substantially a hydrocarbon group of up to 20 carbon atoms, and X is an alkyl or cycloalkyl group of up to 10 carbon atoms, provided that -CO-X is not enolisable, which comprises the asymmetric epoxidation of a corresponding prochiral alkene of formula II
R¹R²C=CR³-CO-X (II)
by reaction with an oxidant in the presence of a chiral catalyst.

2. A process according to claim 1, wherein the catalyst is a heterogeneous chiral polymer.

3. A process according to claim 1, wherein the catalyst is a metal-free synthetic polypeptide.

4. A process according to claim 1, wherein the catalyst is obtainable by nucleophile-promoted oligomerisation of an amino-acid carboxy anhydride.

5. A process according to claim 4, wherein the anhydride has the partial formula

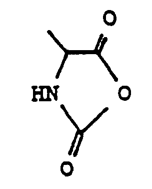

6. A process according to claim 5, wherein the oligomerisation is caused by humidity or an amine.

7. A process according to any preceding claim, which is conducted in the presence of alkali such as hydroxide.

8. A process according to any of claims 2 to 6, which is conducted in a three-phase mixture of the polymer or oligomer, an organic solvent, and an aqueous phase containing the oxidant and alkali, in the presence of a catalytic amount of a phase-transfer catalyst such as Aliquat-336.

9. A process according to claim 7 or claim 8, wherein not more than one equivalent of alkali is used.

10. A process according to any preceding claim, wherein the oxidant is a perborate.

11. A process according to any preceding claim, wherein R² and R³ are each H.

12. A process according to claim 11, wherein R¹ is H.

13. A process according to any of claims 1 to 11, wherein R¹ is a group of up to 10 carbon atoms.

14. A process according to any preceding claim, wherein X is t-butyl.

15. A process according to claim 14, for the preparation of (1*S*,2*R*)-trans-1,2-epoxy-4,4-dimethyl-1-phenyl-3-pentanone from (*E*)-4,4-dimethyl-1-phenylpenten-3-one.

16. An optically-enriched chiral epoxide of formula I as defined in claim 1, in more than 80% enantiomeric excess, wherein R¹ is aryl or heteroaryl, optionally linked *via* a epoxide group to CR².

17. An epoxide according to claim 16, wherein R¹ is optionally-substituted phenyl.

18. An epoxide according to claim 16, wherein R¹ is phenyl.

19. An epoxide according to any of claims 16 to 18, wherein X is t-butyl or cyclopropyl.

20. An epoxide according to claim 19, wherein X is t-butyl.

21. An epoxide according to any of claims 16 to 18, wherein X is t-alkyl.

22. A process for the preparation of an optically-enriched epoxide ester, which comprises conducting a process according to any of claims 1 to 15, and then converting X in the epoxide to OX, by the Baeyer-Villiger reaction.

23. A process according to claim 22, wherein OX is t-butoxy.

24. Use of (1*S*,2*R*)-*trans*-1,2-epoxy-4,4-dimethyl-1-phenyl-3-pentanone for the preparation of syn-3-phenylisoserine or a derivative thereof represented by the formula 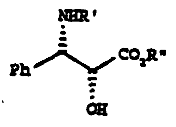 enriched in the (2*R*,3*S*)-stereoisomer, wherein R' is H or acyl and R" is H or alkyl.

25. Use according to claim 24, wherein R' is benzoyl.

26. Use according to claim 24, which comprises:
(i) Baeyer-Villiger oxidation to produce (2*R*,3*S*)-*t*-butyl 2,3-epoxy-3-phenylpropanoate;
(ii) inversion of configuration at C-3 to afford the corresponding *cis*-epoxide;
(iii) nucleophilic ring opening at the benzylic position with either ammonia or azide anion (followed by reduction to the amine); and
(iv) N-benzoylation and, optionally, acid-catalysed deesterification.

27. Use according to claim 24, which comprises:
(i) conversion to an intermediate of formula 7; and
(ii) Baeyer-Villiger oxidation to give the product wherein R" is H or *t*-butyl and, optionally, acid-catalysed deesterification.

28. An epoxide according to claim 17, wherein R¹ is 4-methoxyphenyl.

29. A process for the preparation of diltiazem, which comprises conducting a process according to claim 13 when R¹ is 4-methoxyphenyl, and converting the product to diltiazem.

## Patentansprüche

1. Verfahren zur Herstellung eines optisch angereicherten chiralen Epoxids der Formel I 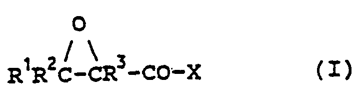 worin R¹, R² und R³ jeweils unabhängig ausgewählt sind aus H, R, R-CO- und R-O-CO-, wobei jedes R unabhängig im wesentlichen eine Kohlenwasserstoffgruppe mit bis zu 20 Kohlenstoffatomen bedeutet und X eine Alkyl- oder Cycloalkylgruppe mit bis zu 10 Kohlenstoffatomen bedeutet, mit der Maßgabe, daß -CO-X nicht enolisierbar ist, welches die asymmetrische Epoxidierung eines entsprechenden prochiralen Alkens der Formel II
R¹R²C=CR³-CO-X (II)
durch Reaktion mit einem Oxidationsmittel in Gegenwart eines chiralen Katalysators umfaßt.

2. Verfahren nach Anspruch 1, worin der Katalysator ein heterogenes chirales Polymer ist.

3. Verfahren nach Anspruch 1, worin der Katalysator ein metallfreies synthetisches Polypeptid ist.

4. Verfahren nach Anspruch 1, worin der Katalysator erhältlich ist durch Nukleophil-geförderte Oligomerisation eines Aminosäurecarboxyanhydrids.

5. Verfahren nach Anspruch 4, worin das Anhydrid die Teilformel 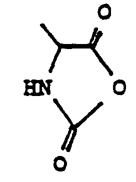 aufweist.

6. Verfahren nach Anspruch 5, worin die Oligomerisation durch Feuchtigkeit oder ein Amin bewirkt wird.

7. Verfahren nach irgendeinem vorhergehenden Anspruch, welches in Gegenwart von Alkali, z.B. Hydroxid, durchgeführt wird.

8. Verfahren nach irgendeinem der Ansprüche 2 bis 6, welches in einer dreiphasigen Mischung des Polymers oder Oligomers, eines organischen Lösungsmittels und einer wässrigen Phase, die das Oxidationsmittel und Alkali enthält, in Gegenwart einer katalytischen Menge eines Phasentransfer-Katalysators, wie z.B. Aliquat-336, durchgeführt wird.

9. Verfahren nach Anspruch 7 oder Anspruch 8, worin nicht mehr als ein Äquivalent Alkali verwendet wird.

10. Verfahren nach irgendeinem vorhergehenden Anspruch, worin das Oxidationsmittel ein Perborat ist.

11. Verfahren nach irgendeinem vorhergehenden Anspruch, worin R² und R³ jeweils H bedeuten.

12. Verfahren nach Anspruch 11, worin R¹ H ist.

13. Verfahren nach irgendeinem der Ansprüche 1 bis 11, worin R¹ eine Gruppe mit bis zu 10 Kohlenstoffatomen bedeutet.

14. Verfahren nach irgendeinem vorhergehenden Anspruch, worin X t-Butyl ist.

15. Verfahren nach Anspruch 14 zur Herstellung von (1S,2R)-trans-1,2-Epoxy-4,4-dimethyl-1-phenyl-3-pentanon aus (E)-4,4-Dimethyl-1-phenylpenten-3-on.

16. Optisch angereichertes chirales Epoxid der Formel 1, wie in Anspruch 1 definiert, in mehr als 80% enantiomerem Überschuß, worin R¹ ein gegebenenfalls über eine Epoxidgruppe an CR² gebundenes Aryl oder Heteroaryl bedeutet.

17. Epoxid nach Anspruch 16, worin R¹ für gegebenenfalls substituiertes Phenyl steht.

18. Epoxid nach Anspruch 16, worin R¹ Phenyl bedeutet.

19. Epoxid nach irgendeinem der Ansprüche 16 bis 18, worin X t-Butyl oder Cyclopropyl bedeutet.

20. Epoxid nach Anspruch 19, worin X t-Butyl ist.

21. Epoxid nach irgendeinem der Ansprüche 16 bis 18, worin X t-Alkyl ist.

22. Verfahren zur Herstellung eines optisch angereicherten Epoxidesters, welches umfaßt das Durchführen eines Verfahrens nach irgendeinem der Ansprüche 1 bis 15 und das anschließende Überführen von X in dem Epoxid in OX mit Hilfe der Baeyer-Vllliger-Reaktion.

23. Verfahren nach Anspruch 22, worin OX t-Butoxy ist.

24. Verwendung von (1S,2R)-trans-1,2-Epoxy-4,4-dimethyl-1-phenyl-3-pentanon zur Herstellung von an (2R,3S)-Stereoisomer angereichertem syn-3-Phenylisoserin oder einem Derivat davon, dargestellt durch die folgende Formel 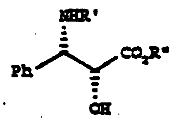 worin R' für H oder Acyl steht und R" für H oder Alkyl steht.

25. Verwendung nach Anspruch 24, worin R' Benzoyl bedeutet.

26. Verwendung nach Anspruch 24, umfassend:
(i) Baeyer-Villiger-Oxidation zur Herstellung von (2R,3S)-t-Butyl-2,3-epoxy-3-phenylpropanoat;
(ii) Inversion der Konfiguration an C-3, um das entsprechende cis-Epoxid bereitzustellen;
(iii) nukleophile Ringöffnung an der Benzyl-Position mit entweder Ammoniak oder Azid-Anion (gefolgt von Reduktion zu dem Amin); und
(iv) N-Benzoylierung und gegebenenfalls Säure-katalysierte Esterspaltung.

27. Verwendung nach Anspruch 24, umfassend:
(i) Überführen in ein Zwischenprodukt der Formel 7; und
(ii) Baeyer-Villiger-Oxidation, um das Produkt zu ergeben, worin R" H oder t-Butyl bedeutet, und gegebenenfalls Säure-katalysierte Esterspaltung.

28. Epoxid nach Anspruch 17, worin R¹ 4-Methoxyphenyl ist.

29. Verfahren zur Herstellung von Diltiazem, umfassend das Durchführen eines Verfahrens nach Anspruch 13, wenn R¹ für 4-Methoxyphenyl steht, und das Überführen des Produkts in Diltiazem.

## Revendications

1. Procédé pour la préparation d'un époxyde chiral optiquement enrichi de formule I 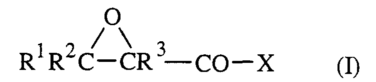 dans laquelle R¹, R² et R³ sont chacun choisis, de façon indépendante, parmi H, R, R-CO- et R-O-CO-, chaque R étant, de façon indépendante, substantiellement un groupe hydrocarboné ayant jusqu'à 20 atomes de carbone et X étant un groupe alkyle ou cycloalkyle ayant jusqu'à 10 atomes de carbone, sous réserve que -CO-X ne soit pas énolisable, qui comprend l'époxydation asymétrique d'un alcène prochiral correspondant de formule II
R¹R²C=CR³―CO―X (II)
par réaction avec un oxydant en présence d'un catalyseur chiral.

2. Procédé selon la revendication 1, dans lequel le catalyseur est un polymère chiral hétérogène.

3. Procédé selon la revendication 1, dans lequel le catalyseur est un polypeptide de synthèse exempt de métal.

4. Procédé selon la revendication 1, dans lequel le catalyseur peut être obtenu par une oligomérisation d'un carboxy anhydride d'un aminoacide favorisée par un nucléophile.

5. Procédé selon la revendication 4, dans lequel l'anhydride a pour formule partielle

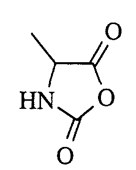

6. Procédé selon la revendication 5, dans lequel l'oligomérisation est induite par l'humidité ou par une amine.

7. Procédé selon une quelconque des revendications précédentes, qui est réalisé en présence d'une base telle qu'un hydroxyde.

8. Procédé selon une quelconque des revendications 2 à 6, qui est réalisé dans un mélange à trois phases constitué par le polymère ou l'oligomère, un solvant organique et une phase aqueuse contenant l'oxydant et une base, en présence d'une quantité catalytique d'un catalyseur de transfert de phase tel que l'Aliquat-336.

9. Procédé selon la revendication 7 ou la revendication 8, dans lequel on n'utilise pas plus d'un équivalent de base.

10. Procédé selon une quelconque des revendications précédentes, dans lequel l'oxydant est un perborate.

11. Procédé selon une quelconque des revendications précédentes, dans lequel R² et R³ sont chacun H.

12. Procédé selon la revendication 11, dans lequel R¹ est H.

13. Procédé selon une quelconque des revendications 1 à 11, dans lequel R¹ est un groupe ayant jusqu'à 10 atomes de carbone.

14. Procédé selon une quelconque des revendications précédentes, dans lequel X est le t-butyle.

15. Procédé selon la revendication 14, pour la préparation de la (*1S*,*2R*)-*trans*-1,2-époxy-4,4-diméthyl-1-phényl-3-pentanone à partir de la (*E*)-4,4-diméthyl-1-phényl-pentén-3-one.

16. Epoxyde chiral optiquement enrichi de formule I tel que défini dans la revendication 1, dans lequel un énantiomère est en excès à plus de 80 % et dans lequel R¹ est un groupe aryle ou un hétéroaryle, éventuellement lié par l'intermédiaire d'un groupe époxyde à CR².

17. Epoxyde selon la revendication 16, dans lequel R¹ est un phényle éventuellement substitué.

18. Epoxyde selon la revendication 16, dans lequel R¹ est le phényle.

19. Epoxyde selon une quelconque des revendications 16 à 18, dans lequel X est le t-butyle ou le cyclopropyle.

20. Epoxyde selon la revendication 19, dans lequel X est le t-butyle.

21. Epoxyde selon une quelconque des revendications 16 à 18, dans lequel X est un t-alkyle.

22. Procédé pour la préparation d'un ester d'époxyde optiquement enrichi, qui comprend la réalisation d'un procédé selon une quelconque des revendications 1 à 15, puis la conversion de X, dans l'époxyde, en OX, par la réaction de Baeyer - Villiger.

23. Procédé selon la revendication 22, dans lequel OX est le t-butoxy.

24. Utilisation de la (*1S*,*2R*)*-trans*-1,2-époxy-4,4-diméthyl-1-phényl-3-pentanone pour la préparation de la *syn*-3-phénylisosérine ou d'un de ses dérivés représentés par la formule 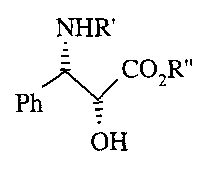 enrichis en stéréoisomère (*2R*,*3S*), où R' est un H ou un acyle et R" est un H ou un alkyle.

25. Utilisation selon la revendication 24, dans laquelle R' est le benzoyle.

26. Utilisation selon la revendication 24, qui comprend :
(i) une oxydation de Baeyer - Villiger pour produire le (*2R*,*3S*)-2,3-époxy-3-phénylpropanoate de t-butyle ;
(ii) l'inversion de la configuration en C-3 pour obtenir le *cis*-époxyde correspondant ;
(iii) l'ouverture nucléophile du cycle en position benzylique par de l'ammoniac ou un anion azoture (suivie de la réduction en amine) ; et
(iv) une N-benzoylation et, éventuellement, une désestérification catalysée par un acide.

27. Utilisation selon la revendication 24, qui comprend :
(i) la conversion en un intermédiaire de formule **7** ; et
(ii) une oxydation de Baeyer - Villiger pour donner le produit dans lequel R" est H ou le t-butyle et, éventuellement, une désestérification catalysée par un acide.

28. Epoxyde selon la revendication 17, dans lequel R¹ est le 4-méthoxyphényle.

29. Procédé pour la production du diltiazem, qui comprend la réalisation d'un procédé selon la revendication 13, quand R¹ est le 4-méthoxyphényle, et la conversion du produit en diltiazem.
